(19) Europäisches Patentamt
European Patent Office
Office européen des brevets

(11) Publication number: **0 381 335 A1**

# (12) EUROPEAN PATENT APPLICATION

(21) Application number: **90300491.9**

(22) Date of filing: **17.01.90**

(51) Int. Cl.[5]: **C07H 19/073, C07H 19/173, A61K 31/70**

A request for correction of claim 1, 7 and 9 has been filed pursuant to Rule 88 EPC. A decision on the request will be taken during the proceedings before the Examining Division (Guidelines for Examination in the EPO, A-V, 2.2).

(30) Priority: **31.01.89 GB 8902041**

(43) Date of publication of application: **08.08.90 Bulletin 90/32**

(84) Designated Contracting States: **DE FR GB IT**

(71) Applicant: **TANABE SEIYAKU CO., LTD.**
**2-10, Dosho-machi 3-chome**
**Chuo-ku Osaka(JP)**

(72) Inventor: **De Clercq, Erik**
**Minderbroedersstraat 10**
**B-3000 Leuven(BE)**
Inventor: **Inoue, Ichizo**
**No. 2-11, Suimeidai 3-chome**
**Kawanishi-shi, Hyogo-ken(JP)**
Inventor: **Kondo, Kazuhiko**
**No. 8-1-810, Utajima 3-chome,**
**Nishiyodogawa-ku**
**Osaka-shi, Osaka-fu(JP)**

(74) Representative: **Hardisty, David Robert et al**
**BOULT, WADE & TENNANT 27 Furnival Street**
**London EC4A IPQ(GB)**

## (54) Antiviral agent and novel nucleoside.

(57) There are disclosed an antiviral agent which comprises a nucleoside of the formula:

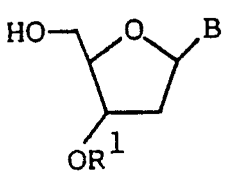

(I)

wherein B is a thymin-1-yl group, a cytosin-1-yl group, an adenin-9-yl group or a guanin-9-yl group; and $R^1$ is amino group when B being a thymin-1-yl group, a cytosin-1-yl group, an adenin-9-yl group or a guanin-9-yl group, -NHCHO when B being a thymin-1-yl group or an adenin-9-yl group, $-N=CH_2$ when B being a thymin-1-yl group, or $-N=C(CH_3)_2$ when B being a thymin-1-yl group or an adenin-9-yl group, or a pharmaceutically acceptable salt thereof as an active therapeutic substance, and a pharmaceutical composition which comprises the nucleoside as mentioned above and a pharmaceutically.acceptable carrier, and a novel nucleoside used therefor.

EP 0 381 335 A1

# Antiviral agent and novel nucleoside

## BACKGROUND OF THE INVENTION

The present invention relates to a pharmaceutical compound, particularly to a compound represented by the formula:

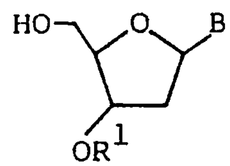

(I)

wherein B is a thymin-1-yl group, a cytosin-1-yl group, an adenin-9-yl group or a guanin-9-yl group; and $R^1$ is amino group when B being a thymin-1-yl group, a cytosin-1-yl group, an adenin-9-yl group or a guanin-9-yl group, -NHCHO when B being a thymin-1-yl group or an adenin-9-yl group, $-N=CH_2$ when B being a thymin-1-yl group, or $-N=C(CH_3)_2$ when B being a thymin-1-yl group or an adenin-9-yl group, or a pharmaceutically acceptable salt thereof which is useful as antiviral agent.

It is described in Nucleic Acids Symposium Series, No. 16, pp. 93 - 96 (1985) that 3′-O-aminothymidine can be synthesized from 1-(5-O-trityl-2-deoxy-β-D-*threo*-pentofuranosyl)thymine, and also 3′-O-amino-2′-deoxycytidine from 1-(5-O-trityl-2-deoxy-β-D-*threo*-pentofuranosyl)uracil. However, nothing is known about pharmaceutical action of these compounds.

## SUMMARY OF THE INVENTION

The present inventors have studied intensively and consequently found that 3′-O-substituted thymidine, 3′-O-amino-2′-deoxycytidine, 3′-O-substituted-2′-deoxyadenosine and 3′-O-amino-2′-deoxyguanosine have activities against human retrovirus, particularly against human immunodeficiency virus (hereinafter abbreviated as HIV), and are useful as pharmaceutical compounds.

That is, an antiviral agent of the present invention comprises a nucleoside represented by the following formula (I):

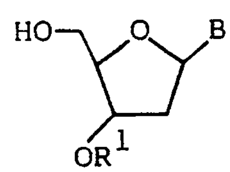

(I)

wherein B is a thymin-1-yl group, a cytosin-1-yl group, an adenin-9-yl group or a guanin-9-yl group; and $R^1$ is amino group when B being a thymin-1-yl group, a cytosin-1-yl group, an adenin-9-yl group or a guanin-9-yl group, -NHCHO when B being a thymin-1-yl group or an adenin-9-yl group, $-N=CH_2$ when B being a thymin-1-yl group, or $-N=C(CH_3)_2$ when B being a thymin-1-yl group or an adenin-9-yl group, or a pharmaceutically acceptable salt thereof.

## DESCRIPTION OF THE PREFERRED EMBODIMENTS

The anti-HIV activity of the agents that are subject of the present invention was measured according to the method of E. De Clercq et al. (The Journal of Virological Methods, 16 : 171 - 185 (1987) and The Journal of Virological Methods, 20 : 309-321 (1988)). The effective amount of 3′-O-amino-2′-deoxycytidine necessary for permitting the cells infected with HIV to live to the 5th day after infection ($ED_{50}$) was found to

be 61 µM, whereas at the concentration of 500 µM, no cytotoxicity appeared. Also, when the anti-HIV activity was measured for 3′-O-aminothymidine, $ED_{50}$ was found to be 23 µM, and only slight cytotoxicity appeared at the concentration of 125 µM.

When the anti-HIV activity was measured for 3′-O-amino-2′-deoxy adenosine, $ED_{50}$ was 32 µM, whereas no cytotoxicity was observed up to a concentration of 1000 µM.

HIV has been known as a pathogen for AIDS, infects selectively T-cells having OKT4 surface marker to disturb the overall balance of the immune system and also to reduce the cell's defense against infection. Accordingly, 3′-O-substituted thymidine, 3′-O-amino-2′-deoxycytidine, 3′-O-substituted-2′-deoxyadenosine and 3′-O-amino-2′-deoxyguanosine or a pharmaceutically acceptable salt thereof can be used for therapy of an AIDS patient showing opportunistic infections. Also, recently, HIV has been found in B cells exhibiting T4 marker, cells of the central nervous system, macrophages and other tissue species including non-blood related tissues, and HIV is known to bring about symptoms such as encephalopathy associated with progressive demyelinating diseases, ataxia and disorientation, and the above compounds of the present invention can be also used for therapy of such various symptoms. Further, the compounds of the present invention can be used for other various symptoms accompanied with HIV infection, such as symptomless carrier state, progressive generalized lymphadenopathy (PGL) and AIDS-related complex (ARC).

3′-O-Substituted thymidine, 3′-O-amino-2′-deoxycytidine, 3′-O-substituted-2′-deoxyadenosine and 3′-O-amino-2′-deoxyguanosine or a parmaceutically acceptable salt thereof can be used by either oral administration or parenteral admini stration. When used by oral administration, it can be used as such or a pharmaceutical preparation together with a pharmaceutical carrier suitable for oral administration such as excipient, binder, disintegrating agent, lubricant, etc. Examples of such pharmaceutical carrier may include, starch powder, lactose, glucose, gelatin, sorbitol, tragacanth, polyvinyl pyrrolidone, lactose, sucrose, corn starch, polyethylene glycol, talc, potassium phosphate, magnesium stearate, and other excipients, binders, disintegrating agents, lubricants, etc. as suitable ones. Also, the dosage form may be a solid agent such as tablet, capsule, granule, microcapsule and suppository, or alternatively a liquid agent such as solution, suspension and emulsion. On the other hand, when aimed for parenteral administration, the compound of the present invention should preferably be used as an injection agent, and the solvent to be used for this purpose may be conveniently, for example, distilled water for injection, a vegetable oil, propylene glycol, etc., and further safe dissolving aids, buffering agent, stabilizers, etc. may be included.

Examples of the pharmaceutically acceptable salts of the compounds of the present invention may be, for example, acid addition salts such as hydrochloride, sulfate, phosphate, sulfamidate, acetate, lactate, tartrate, maleate, succinate, methanesulfonate, ethanesulfonate, benzenesulfonate and p-toluenesulfonate.

The dose of 3′-O-substituted thymidine, 3′-O-amino-2′-deoxycytidine, 3′-O-substituted-2′-deoxyadenosine and 3′-O-amino-2′-deoxyguanosine or pharmaceutically acceptable salts thereof, which may also depend on the kind of disease, the age and the body weight of patient, severity of disease and administration route, etc., may appropriately be comprised between 0.1 and 100 mg/kg, preferably 1 to 10 mg/kg, for human adult per day.

## EXAMPLES

Anti-HIV activity of the compounds was determined by the following methods.

1. Trypan Blue exclusion method and indirect immuno fluorescence method combined with laser cytofluorometry

### Virus growth and partial purification

HIV was obtained from the supernatant of a persistently HIV-infected H9 cell line (H9/HTLV-$III_B$).

Clone H9 is an $OKT4^+$ T-cell line that is permissive for HIV replication but partially resistant to the cytopathic effect (CPE) of the virus.

H9/HTLV-$III_B$ cells were grown in 75-$cm^2$ loosely capped cell culture flasks (Nunc, Kamstrup, Roskilde, Denmark) in RPMI 1640 DM ('Dutch modification') medium (containing 20 m Hepes) (Flow-Laboratories, Irvine, Scotland), supplemented with 10 % (v/v) heat inactivated fetal calf serum (Gibco Biocult, Glasgow, Scotland), 2,5 µg/ml amphotericin B and 20 µg/ml gentamycin.

The cells were maintained at 37 °C in a humidified atmosphere of 5 % $CO_2$. Every 3 to 4 days, cells were spun down and seeded at 500,000 cells/ml in new cell culture flasks. The cell-free culture fluids were pooled for virus purification and kept frozen at - 70 °C.

For virus purification, the cell supernatants were filtered through a 0.22 µm Millipore filter (Millipore Corporation, Bedford, Massachusetts). Virus was subsequently pelleted in a Beckman ultracentrifuge (model L3-50) at 4 °C (SW 27 rotor at 26,000 rpm for 2 hours). The viral pellets were resuspended in RPMI Medium (1/30 of the original volume) and stored at - 70 °C before use.

The virus stock used in our experiments had a titer of $10^{3.7}$ $CCID_{50}$ ml (1 $CCID_{50}$ being the 50 % cell culture infective dose).

T-cells

The HTLV-I infected MT-4 cell line was established by co-cultivating leukocytes from an adult T-cell leukemia (ATL) patient with cord blood leukocytes. The cells were grown and maintained under the same conditions as the H9/HTLV-$III_B$ cell line.

Assay of cytophatic effect (CPE) of HIV in MT-4 cells

MT-4 cells of a 24 hours-old culture were suspended at 5 x $10^5$ cells per ml. Of this suspension, 2-ml portions were divided over polyisopropylene 2059 Falcon culture tubes (Becton Dickinson). The MT-4 cell suspensions were then centrifuged for 10 min at 140 x g and the supernatants were discarded. The MT-4 cell pellets were infected with 200 µl of a 1:5 HIV stock solution (titer $10^3$ $CCID_{50}$ ml; i.e., finally 10 $CCID_{50}$ well) in RPMI medium (composition as described above). Control MT-4 cell pellets were mock-infected with 200 µl RPMI medium. The culture tubes were then capsulated and subsequently incubated at 37 °C for 1.5 hours, upon which 1.8 ml of RPMI medium was added to each tube. The HIV-infected and mock-infected MT-4 cells were each pooled in two 45-ml centrifuge tubes.

Varying dilutions of the test compounds in RPMI medium were added in 100-µl volumes to the wells of a flat bottom, 96-well plastic microtiter tray (Sterilin Ltd. Teddington, Middlesex, England).

Of the HIV-infected or mock-infected MT-4 cell suspensions 100-µl volumes were added to the wells. The cell cultures were then incubated at 37 °C in a 5 % $CO_2$/95 % air humidified atmosphere. The cells remained in contact with the test compounds during the whole incubation period. Five days after infection the viability of the cells was examined microscopically in a hematocytometer by the trypan blue dye exclusion method.

Alternatively, viability of the cells was determined by the tetrazolium-based colorimetric method (MTT method) as described in the Journal of Virological Methods, 20 : 309 - 321 (1988).

The results are shown in Table 1.

Determination of HIV antigen expression by indirect immunofluorescence

MT-4 cells in microtray wells were infected with HIV and exposed to varying concentrations of the compounds as described above for the cytopathic effect assay.

MT-4 cells of two wells were pooled in Falcon 2054 tubes (Becton Dickinson) and washed twice in PBS by centrifugation at 160 x g for 10 min. Subsequently, the MT-4 cell pellet was incubated at 37 °C for 45 min in the presence of 50 µl anti-HIV serum (diluted 1:1000 in PBS), which was obtained from a Zairian patient. The washing procedure in PBS was repeated twice and the samples were incubated at 37 °C for 45 min with 50 µl of goat anti-human IgG conjugated with fluorescein isothiocyanate (diluted 1:40 in PBS). Following two washing steps with PBS the cells were fixed with 1 ml of 0.37 % formaldehyde (Vel, Aalst, Belgium) in PBS and stored in the dark at 4 °C. Mock-infect ed MT-4 cells, treated in a similar fashion, served as the controls. Cytofluorographic analyses were performed within one week following sample preparation.

The flow cytofluorometer used was a cytofluorograph 30-L (Ortho Diagnostic Instruments) equipped with a dual laser system. For measurements of the axial light extinction, which correlates with cell size, we used an 0.8-mW helium-neon laser (Lexel Corporation, Palo Alto, California). A low-power argon ion laser (Model 75, Lexel Corp.) was used for fluorescence excitation. The excitation wavelength was 488 nm. In all experiments, the laser power was 100 mW. The cytofluorograph was interfaced to a Model 2150 Computer

System (Ortho Diagnostic Systems), allowing data on the intensity of green fluorescence (pulse height) of each cell to be displayed and printed as histograms. Each histogram was based on the analysis of 10,000 cells. The data were analyzed quantitatively based on the percentage of fluorescent cells in each sample. The window facility together with the histogram pattern of the mock-infected MT-4 cells were used to determine a minimum fluorescence intensity threshold value, and to eliminate cellular autofluorescence from our data.

The results are shown in Table 1.

Table 1

(1) 3'-O-aminothymidine

| | A | | B | |
|---|---|---|---|---|
| Concentration (μM) | Cell viability (Trypan Blue exclusion) HIV-infected | Mock-infected | Immunofluorocytography (%) | Inhibition** (%) |
| 125 | 78 *1 | 77 *2 | 9.5 | 70 *3 |
| 25 | 61 (53) | 128 | 7.7 | 76 |
| 5 | 3 (3) | 126 | 22.6 | 30 |
| 1 | 2 | 128 | 30.6 | 5 |
| 0 (Control) | 1 | 115 | 32.2 | |

*1 $ED_{50}$ = 22.7 μM
*2 $TD_{50}$ > 125 μM
*3 $ED_{50}$ ~ 10 μM

(2) 3'-O-amino-2'-deoxycytidine

| | A | | B | |
|---|---|---|---|---|
| Concentration (μM) | Cell viability (Trypan Blue exclusion) HIV-infected | Mock-infected | Immunofluorocytography (%) | Inhibition* (%) |
| 500 | 85 *4 | 110 *5 | 7.5 | 74 *6 |
| 125 | 87 (65) | 104 | 9.0 | 68 |
| 25 | 41 (31) | 124 | 15.6 | 45 |
| 5 | 1 | 114 | 26.9 | 6 |
| 0 (Control) | 1 | 134 | 28.5 | |

*4 $ED_{50}$ = 61.5 μM
*5 $TD_{50}$ > 500 μM
*6 $ED_{50}$ ~ 20 μM

$$** \ 100 - 100 \times \frac{\text{Number of fluorescent cells in the presence of test compound (\%)}}{\text{Number of fluorescent cells in the absence of test compound (\%)}}$$

(3) 3'-O-amino-2'-deoxyadenosine

$ED_{50}$ = 32 μM $TD_{50}$ = 1000 μM
both as determined by the MTT method in MT-4 cells
$ED_{50}$ = 58 μM, as based on immunofluorocytography in HUT-78 cells

Cell cultures

The HTLV-I infected MT-4 cell line was established by co-cultivating leukocytes from an adult T-cell leukemia (ATL) patient with cord blood leukocytes (CBL) (Miyoshi et al., 1981) and was kindly provided by Dr. N. Yamamoto, Yamaguchi University, Yamaguchi, Japan. The MT-4 cells were grown in RPMI 1640 DM ('Dutch modification') medium (Flow Laboratories, Irvine, Scotland), supplemented with 10 % (v/v) heat-inactivated fetal calf serum (FCS) and 20 μg/ml gentamycin (E. Merck, Darmstadt, F.R.G.). The cells were maintained at 37 °C in a humidified atmosphere of 5 % $CO_2$ in air. Every 3-4 days, cells were spun down and seeded at 2 x $10^5$ cells/ml in new cell culture flasks. At regular time intervals, the MT-4 cells were analyzed for the presence of mycoplasma and consistently found to be mycoplasma-free.

Virus

HIV (strain HTLV-$III_B$) which was kindly provided by Dr. R.C. Gallo (National Cancer Institute, Bethesda, MD) was obtained from the culture supernatant of a persistently HIV-infected HUT-78 cell line (HUT-78/HTLV-$III_B$). The virus titer of the supernatent was determined in MT-4 cells. The virus stock [2.4 x $10^4$ $CCID_{50}$ (50 % cell culture infective dose) per ml] was stored at - 70 °C until used.

Compounds

O-Substituted nucleosides were synthesized according to the following preparation examples. Stock solutions were made in phosphate-buffered saline (PBS) and stored at 4 °C. Prior to their addition to the microtiter tray wells, the compounds were diluted in RPMI 1640 DM medium at a 10-fold higher concentration than the highest final test concentration.

Anti-HIV assay

Flat bottom, 96-well plastic mictrotiter trays (Falcon, Becton Dickinson, Mountain View, CA) were filled with 100 μl of complete medium using a Titertek® Multidrop dispenser (Flow Laboratories). This eight-channel dispenser could fill a microtiter tray in less than 10 s. Subsequently, stock solutions (10 x final test concentration) of compounds were added in 25 μl volumes to two series of triplicate wells so as to allow simultaneous evaluation of their effects on HIV- and mock-infected cells. Serial five-fold dilutions were made directly in the microtiter trays using an eight-channel Titertek® pipette (FLow Laboratories). Untreated control HIV- and mock-infected cell samples were included for each compound.

Exponentially growing MT-4 cells were centrifuged for 10 min at 140 x g and the supernatants were discarded. The pellet was either infected with 100 $CCID_{50}$ or mock-infected. The MT-4 cells were resuspended at 4 x $10^5$ cells/ml in two flasks which were connected with an autoclavable dispensing cassette of a Titertek® Multidrop dispenser. Under slight magnetic stirring 100-μl volumes were then transferred to the microtiter tray wells. The outer row wells were filled with 100 μl of medium. The cell cultures were incubated at 37 °C in a humidified atmosphere of 5 % $CO_2$ in air. The cells remained in contact with the test compounds during the whole incubation period. Five days after infection the viability of mock- and HIV infected cells was examined either microscopically in a hemacytometer by the trypan blue exclusion method or spectrophotometrically by the MTT method.

MTT method

The MTT method is based on the reduction of the yellow coloured 3-(4,5-dimethylthiazol-2-yl)-2,5-diphenyltetrazolium bromide (MTT) (Sigma Chemical Co., St. Louis, MO) by mitochondrial dehydrogenases of metabolically active cells to a blue formazan which can be measured spectrophotometrically. Therefore, to each well of the microtiter trays, 20 µl of a solution of MTT (7.5 mg/ml) in phosphate-buffered saline was added using the Titertek® Multidrop. The trays were further incubated at 37 °C in a $CO_2$ incubator for the indicated times. A fixed volume of medium (150 µl) was then removed from each cup using an eight-channel pipette without disturbing the MT-4 cell clusters containing the formazan crystals. If necessary, this step was preceded by centrifugation of the microtiter trays (450 x g, 5 min) in a plate holder (8 trays per run).

Solubilization of the formazan crystals was achieved by adding 100 µl 10 % (v/v) Triton X-100 in acidified isopropanol (2 ml concentrated HCl per 500 ml solvent) using the Titertek® Multidrop. Complete dissolution of the formazan crystals could be obtained after the trays had been placed on a plate shaker for 10 min (Flow Laboratories). Finally, the absorbances were read in an eight-channel computer-controlled photometer (Multiskan MCC, Flow Laboratories) at two wavelengths (540 and 690 nm). The absorbance measured at 690 nm was automatically subtracted from the absorbance at 540 nm, so as to eliminate the effects of non-specific absorption. Blanking was carried out directly on the microtiter trays with the first column wells which contained all reagents except for the MT-4 cells. All data represent the average values for a minimum of three wells. The 50 % cytotoxic dose ($CD_{50}$) was defined as the concentration of compound that reduced the absorbance ($OD_{540}$) of the mock-infected control sample by 50 %. The percent protection achieved by the compounds in HIV-infected cells was calculated by the following formula:

$$\frac{(OD_T)_{HIV} - (OD_C)_{HIV}}{(OD_C)_{mock} \quad (OD_C)_{HIV}} \text{ expressed in \%,}$$

whereby $(OD_T)_{HIV}$ is the optical density measured with a given concentration of the test compound in HIV-infected cells; $(OD_C)_{HIV}$ is the optical density measured for the control untreated HIV-infected cells; $(OD_C)_{mock}$ is the optical density measured for the control untreated mock-infected cells; all OD values determined at 540 nm. The dose achieving 50 % protection according to the above formula was defined as the 50 % effective dose ($ED_{50}$).

The results are shown in Table 2.

Table 2

| Sample No. | B | $R^1$ | HIV (µM) | |
|---|---|---|---|---|
| | | | $ED_{50}$ | $TD_{50}$ |
| 1 | thymin-1-yl | -NHCHO | 13 | 144 |
| 2 | thymin-1-yl | $-N=CH_2$ | 17 | 106 |
| 3 | thymin-1-yl | $-N=CMe_2$ | 32 | 313 |
| 4 | adenin-9-yl | $-NH_2$ | 32 | 1000 |
| 5 | guanin-9-yl | $-NH_2$ | 2.17 | 74.03 |
| Me: methyl group | | | | |

Preparation example 1

(1) To a solution of 12 g of 1-(5-O-trityl-2-deoxy-β-D-*threo*-pentofuranosyl)thymine, 4.85 g of N-hydroxyphthalimide and 8 g of triphenylphosphine in 150 ml of tetrahydrofuran were added molecular

sieves (4A), and the mixture was stirred overnight. To the mixture was added 8 g of diethylazodicarboxylate under ice-cooling. The mixture was stirred at room temperature for 30 minutes. Then, to the mixture were added 2.4 g of diethylazodicarboxylate and 4 g of triphenylphosphine. After the reaction was over, molecular sieves (4A) were filtered off and the solvent was evaporated. The residue was purified by silica gel column chromatography (solvent; chloroform : methanol = 40 : 1). Recrystallization from ethanol gave 9.6 g of 3′-O-phthalimido-5′-O-tritylthymidine.
Yield: 61 %,
Melting point: 144 to 146 °C.

(2) The above product (250 mg) and 0.3 ml of butylamine were added into 10 ml of methanol and the mixture was heated under reflux overnight. The solvent was evaporated, and the residue was purified by silica gel chromatography (solvent; chloroform : methanol = 50 : 1) to give 172 mg of 3′-O-amino-5′-O-tritylthymidine as an oily product.
Yield: 82 %.

(3) The above product (3.4 g) was dissolved in a 20 ml of 90 % aqueous trifluoroacetic acid, and the solution was allowed to stand at room temperature for 2 hours. The solvent was evaporated, and the residue was triturated with chloroform. The residue was dissolved in a small amount of water. The solution was applied on a column of a strongly acidic ion exchange resin (Diaion SK-1B, $H^+$ form; produced by Mitsubishi Chemical Industries Ltd.). The column was washed with water, eluted with ammonium hydroxide solution and the solvent was evaporated. The residue was crystallized from water to give 1.2 g of 3′-O-aminothymidine.
Yield: 67 %,
Melting point: 166 to 167 °C.

Preparation example 2

(1) To a solution of 20 g of 1-(5-O-trityl-2-deoxy-β-D-*threo*-pentofuranosyl)uracil, 10.5 g of N-hydroxyphthalimide and 16.7 g of triphenylphosphine in 250 ml of tetrahydrofuran, was added dropwise 11.1 g of diethyl azodicarboxylate under ice-cooling, and the mixture was stirred at room temperature overnight. The solvent was evaporated, and the residue was purified by silica gel column chromatography (solvent; chloroform : methanol = 40 : 1). Recrystallization from ethanol gave 16.2 g of 3′-O-phthalimido-5′-O-trityl-2′-deoxyuridine.
Yield: 62 %,
Melting point: 128 to 132 °C.

(2) The above product (1 g) was dissolved in 20 ml of pyridine. To the solution were added 340 mg of 1,2,4-triazole and 600 mg of p-chlorophenylphosphorodichloridate at room temperature. The mixture was stirred for 3 days at room temperature. The solvent was evaporated, and the residue was dissolved in chloroform. After washing with water and drying, the solution was evaporated and the residue was purified by silica gel chromatography (solvent; benzene : ethyl acetate = 1 : 1). Recrystallization from ethanol gave 880 mg of 1-(3-O-phthalimido-5-O-trityl-2-deoxy-β-D-*erythro*-pentofuranosyl)-4-(1,2,4-triazol-1-yl)-pyrimidin-2(1H)-one.
Yield: 82 %,
Melting point: 225 to 228 °C. (decomp.)

(3) The above product (11.5 g) was suspended in 100 ml of dioxane. To the mixture was added 33 ml of conc. ammonium hydroxide. The mixture was stirred for 1 hour. The lower layer of the reaction mixture separated into two layers was concentrated to dryness. The residue was triturated with water and recrystallized from ethanol to give 6.3 g of 3′-O-amino-5′-O-trityl-2′-deoxycytidine.
Yield: 76 %,
Melting point: 207 to 210 °C.

(4) The above product (550 mg) was dissolved in a 60 % aqueous trifluoroacetic acid and the solvent was removed. The residue was dissolved in water and washed with ether. The aqueous layer was applied on a column of a strongly acidic ion exchange resin (Diaion SK-1B, $H^+$ form, produced by Mitsubishi Chemical Industries Co., Ltd.). The column was washed with water, eluted with 1 N ammoniun hydroxide, and the solvent was evaporated. Recrystallization of the residue from water gave 164 mg of 3′-O-amino-2′-deoxycytidine.
Yield: 73 %,
Melting point: 216 to 219 °C.

Preparation example 3

Synthesis of 3′-O-formamidothymidine

A mixture of 1.8 g of 3′-O-aminothymidine and 0.62 g of acetic formic anhydride in 10 ml of acetic acid was stirred at room temperature for 1.5 hours. The resulting solid was collected by filtration and purified by silica gel column chromatography (solvent; chloroform : methanol = 9 : 1). Recrystallization from ethanol gave 0.64 g of 3′-O-formamidothymidine.
Yield: 32 %,
Melting point: 168 to 170 °C,
NMR ($Me_2SO-d_6$): 7.91 ppm (s, 1H, HCO).

Preparation example 4

Synthesis of 3′-O-methylideneaminothymidine

3′-O-Aminothymidine (1.03 g) was dissolved in 4 ml of 37 % formalin. A crystalline precipitate resulted immediately. To this mixture, 4 ml of water was added and the mixture was stirred at room temperature for 10 minutes. The precipitate was collected by filtration and washed with water. Recrystallization from aqueous ethanol gave 624 mg of 3′-O-methylideneaminothymidine.
Yield: 58 %,
Melting point: 142 to 143 °C,
NMR ($Me_2SO-d_6$): 6.69 (d, 1H, J = 8Hz, N = CH), 7.12 (d, 1H, N = CH).

Preparation example 5

Synthesis of 3′-O-isopropylideneaminothymidine

A solution of 1.29 g of 3′-O-aminothymidine in 50 ml of acetone was heated under reflux for 10 minutes. The solvent was evaporated and the residual solid was recrystallized from ethanol to give 1.2 g of 3′-O-isopropylideneaminothymidine.
Yield: 79 %,
Melting point: 173 to 176 °C,
NMR ($Me_2SO-d_6$):

1.83 (s, 6H, 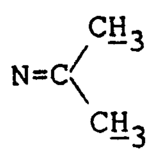 ).

Preparation example 6

(1) Synthesis of 9-[3-O-phthalimido-5-O-(monomethoxytrityl)-2-deoxy-β-D-*erythro*-pentofuranosyl]adenine

To a solution of 3.14 g of 9-[2-deoxy-5-O-(monomethoxy trityl)-β-D-*threo*-pentofuranosyl]adenine, 1.47 g of N-hydroxyphthalimide and 2.36 g of triphenylphosphine in 30 ml of tetrahydrofuran was added a solution of 1.57 g of diethyl azodicarboxylate in 3 ml of tetrahydrofuran under ice-cooling. The mixture was stirred at room temperature for 3 hours and 0.5 ml of water was added. The solvent was evaporated and the residue was dissolved in chloroform. After ice-cooling, crystals deposited were removed by filtration and the filtrate was concentrated to dryness. Purification of the residue by silica gel column chromatography (solvent; chloroform : ethanol = 98 : 2) afforded 1.8 g of 9-[3-O-phthalimido-5-O-(monomethoxytrityl)-2-deoxy-β-D-*erythro*-pentofranosyl]adenine as an amorphous powder.
Yield: 45 %,
NMR ($CDCl_3$): 7.6 - 8.1 (m, 4H, phthaloyl).

(2) Synthesis of 9-(3-O-phthalimido-2-deoxy-β-D-*erythro*-pentofuranosyl)adenine

A solution of the above product (1.67 g) in 20 ml of 90 % aqueous acetic acid was heated at 50 °C for 1 hour. The solvent was removed by evaporation and the residue was crystallized from ethanol to give 0.78 g of 9-(3-O-phthalimido-2-deoxy-β-D-*erythro*-pentofuranosyl)adenine.
Yield: 79 %,
Melting point: 189 to 190 °C,
NMR ($Me_2SO-d_6$ + TFA): 3.6 - 3.9 (m, 2H, $C_5'H_2$).

(3) Synthesis of 9-(3-O-amino-2-deoxy-β-D-*erythro*-pentofuranosyl)adenine

To a solution of the above product (2.3 g) in 30 ml of dioxane was added 50 ml of conc. ammonium hydroxide and the mixture was stirred at room temperature for 2 hours. The mixture was concentrated to a small volume and the insoluble material was removed by filtration. The filtrate was concentrated to dryness and the residue was purified by silica gel column chromatography (solvent; chloroform : methanol = 9 : 1). Recrystallization from aqueous ethanol gave 600 mg of 9-(3-O-amino-2-deoxy-β-D-*erythro*-pentofuranosyl)-adenine.
Yield: 39 %,
Melting point: 254 to 256 °C (decomposed),
NMR ($Me_2SO-d_6$): 4.3 - 4.4 (m, 1H, $C_3'H$), 6.16 (s, 2H, $NH_2$), 7.27 (s, 2H, $NH_2$).

Preparation example 7

Synthesis of 9-(3-O-isopropylideneamino-2-deoxy-β-D-*erythro*-pentofuranosyl)adenine

A mixture of 40 mg of 9-(3-O-amino-2-deoxy-β-D-*erythro*-pentafuranosyl)adenine, 2 ml of acetone and 2 ml of methanol was heated under reflux for 5 minutes. After cooling, crystals deposited were collected by filtration to give 25 mg of 9-(3-O-isopropylideneamino-2-deoxy-β-D-*erythro*-pentafuranosyl)adenine.
Yield: 54 %,
Melting point: 204 to 205 °C,
NMR ($Me_2SO-d_6$):

1.87 (s, 6H, 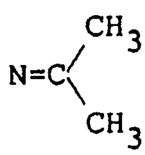 ).

Preparation example 8

Synthesis of 9-(3-O-formamido-2-deoxy-β-D-*erythro*-pentofuranosyl)adenine

To a solution of 100 mg of 9-(3-O-amino-2-deoxy-β-D-*erythro*-pentofuranosyl)adenine in 3 ml of acetic acid was added 0.7 ml of acetic formic anhydride under ice-cooling. The mixture was stirred at room temperature for 15 minutes and the solvent was removed by evaporation. The residue was dissolved in water and passed through a column of Diaion SA-11A (8 ml, $OH^-$). The eluate and the washings were

combined, and evaporated to dryness. The residue was coevaporated with methanol and triturated with ether to give 33 mg of 9-(3-O-formamido-2-deoxy-β-D-*erythro*-pentofuranosyl)adenine as an amorphours powder.
Yield: 30 %,
NMR ($Me_2SO-d_6$): 7.94 (s, 1H, HCO).

Preparation example 9

(1) $N^2$-Isobutyrylguanosine (14 g) was suspended in 140 ml of pyridine, and 14 g of 1,3-dichloro-1,1,3,3-tetraisopropyldisiloxane was added to the suspension and the mixture was stirred at room temperature overnight. After the solvent was evaporated, the residue was dissolved in ethyl acetate and washed successively with water, aqueous citric acid and brine. The organic layer was dried over $MgSO_4$ and evaporated to dryness. The residue was washed with a mixture of ethyl acetate and hexane to give 15.2 g of $N^2$-isobutyryl-3′,5′-O-(1,1,3,3-tetraisopropyldisilox-1,3-diyl)guanosine.
Yield: 64 %,
Melting point: 173 to 175 °C,
NMR ($CDCl_3$): 4.3 - 4.4 (m, 1H, $C_3'H$), 4.5 - 4.6 (m, 1H, $C_2'H$).

(2) The above product (23.5 g) was dissolved in 20 ml of pyridine, and 9 g of p-toluenesulfonyl chloride was added to the solution and the mixture was stirred at room temperature overnight. Ethyl acetate was added to the reaction mixture and the resulting mixture was washed successively with water, aqueous citric acid and brine. The organic layer was dried over $MgSO_4$ and the solvent was evaporated. The residue was purified by silica gel column chromatography to give 23.4 g of $N^2$-isobutyryl-3′,5′-O-(1,1,3,3-tetraisopropyldisilox-1,3-diyl)-2′-O-tosylguanosine.
Yield: 79 %,
Amorphous powder
NMR (δ in $CDCl_3$): 2.42 (s, 3H, ArMe), 5.12 (d, 1H, $C_2'H$), 7.27, 7.77 (d, 4H, ArH).

(3) The above product (0.6 g) was dissolved in 15 ml of tetrahydrofuran, and 1.6 ml of 1M-tetrabutylammonium fluoride was added to the solution and the mixture was stirred at room temperature for 20 minutes. The solvent was evaporated, and chloroform and water were added to the residue. Precipitated crystals were collected by filtration and dried to give 0.37 g of $N^2$-isobutyryl-2′-O-tosylguanosine.
Yield: 74 %,
Melting point: 155 to 160 °C (decomposed),
NMR ($Me_2SO-d_6$): 3.5 - 3.7 (m, 2H, $C_5'H_2$), 4.3 - 4.4 (m, 1H, $C_3'H$).

(4) The above product (10.3 g) was dissolved in 100 ml of pyridine, and 9.4 g of p-anisylchlorodiphenylmethane was added to the solution and the mixture was stirred at room temperature overnight. The solvent was evaporated, and the residue was dissolved in ethyl acetate and the solution was washed successively with water, aqueous citric acid and aqueous sodium bicarbonate. The organic layer was dried and the residue obtained by evaporation was purified by silica gel column chromatography to give 14.5 g of $N^2$-isobutyryl-5′-O-monomethoxytrityl-2′-O-tosylguanosine.
Yield: 92 %,
Amorphous powder
NMR ($CDCl_3$): 3.77 (s, 3H, Me), 7.2 - 7.6 (m, 14H, ArH).

(5) The above product (6 g) was dissolved in 60 ml of ammonia-methanol, and the solution was allowed to stand at room temperature overnight. The solvent was evaporated, and the residue was washed successively with water, methanol and ether. After drying, 4.4 g of 5′-O-monomethoxytrityl-2′-O-tosylguanosine was obtained.
Yield: 81 %,
Melting point: 210 to 212 °C,
NMR ($Me_2SO-d_6$): 6.24 (br s, 2H, $NH_2$).

(6) The above product (4.4 g) was suspended in 120 ml of tetrahydrofuran, and 62 ml of 1M-lithium triethylborohydride was added to the suspension at -10 °C. After stirring at room temperature overnight, 4.5 ml of water was added dropwise to the mixture under ice-cooling and the solvent was evaporated. The residue was purified by silica gel column chromatography to give 3.2 g of 9-[2-deoxy-5-O-(monomethoxytrityl)-β-D-*threo*-pentofuranosyl]guanine.
Yield: 96 %,
Melting point: 194 to 198 °C (decomposed),
NMR ($Me_2SO-d_6$): 2.1 - 2.2, 2.6 - 2.7 (m, 2H, $C_2'H_2$), 4.2 - 4.3 (m, 1H, $C_3'H$).

(7) The above product (0.2 g) was dissolved in 5 ml of dimethylformamide, and 0.31 ml of 1,1,1,3,3,3-hexamethyldisilazane was added to the solution and the mixture was stirred at room temperature for 15 minutes. 0.04 ml of methanol was added to the mixture and the solvent was evaporated. The residue was coevaporated with pyridine, and dissolved in 5 ml of pyridine, and 0.05 ml of isobutyryl chloride was added to the mixture. The mixture was stirred at room temperatrue for 30 minutes. After evaporation of the solvent, the residue was dissolved in ethyl acetate and washed successively with water, aqueous sodium bicarbonate and brine. The organic layer was dried over $MgSO_4$ and the solvent was evaporated. The residue was dissolved in 5 ml of tetrahydrofuran. To the solution was added 0.56 ml of 1M-tetrabutylammonium fluoride, and the mixture was stirred at room temperature for 10 minutes. Ethyl acetate was added to the mixture and the organic layer was washed successively with water and brine. After the organic layer was dried over $MgSO_4$, the solvent was evaporated and the residue was purified by silica gel column chromatography (solvent; $CHCl_3$ : MeOH = 25 : 1) to give 0.17 g of $N^2$-isobutyryl-9-[2-deoxy-5-O-(monomethoxytrityl)-β-D-*threo*-pentofuranosyl]guanine.
Yield: 75 %,
Amorphous powder,
NMR ($CDCl_3$): 1.23 (d, 6H, Me's), 2.5 - 2.8 (m, 3H, $C\underline{H}Me_2$, $C_2{'}H_2$).

(8) The above product (2.2 g), 0.88 g of N-hydroxyphthalimide, 1.4 g of triphenylphosphine and 1 g of Molecular Sieves (4A) were added to 40 ml of tetrahydrofuran, and the mixture was stirred at room temperature for one hour. Under ice-cooling, 0.85 ml of diethyl azodicarboxylate was added to the mixture and the mixture was stirred at room temperature for one hour. Then, 0.44 g of N-hydroxyphthalimide, 0.7 g of triphenylphosphine and 0.43 ml of diethyl azodicarboxylate were added to the mixture and stirring was continued to further one hour. The solvent was evaporated and the residue was purified by silica gel column chromatography (solvent; $CHCl_3$ : MeOH = 100 : 1) to give 0.9 g of $N^2$-isobutyryl-9-[2-deoxy-5-O-(monomethoxytrityl)-3-O-phthalimido-β-D-*erythro*-pentofuranosyl]guanine.
Yield: 33 %,
Amorphous powder,
NMR ($CDCl_3$): 5.2 - 5.3 (m, 1H, $C_3{'}H$), 7.7 - 7.9 (m, 4H, phthaloyl).

(9) The above product (250 mg) was dissolved in 2.5 ml of 90 % aqueous acetic acid and the solution was stirred at room temperature overnight. The solvent was evaporated and the residue was purified by silica gel column chromatography to give 0.09 g of $N^2$-isobutyryl-9-[2-deoxy-3-O-phthalimido-β-D-*erythro*-pentofuranosyl]guanine.
Yield: 56 %,
Amorphous powder,
NMR ($Me_2SO$-$d_6$): 5.1 - 5.2 (m, 2H, $C_3{'}H$, $C_5{'}OH$).

(10) The above product (80 mg) was dissolved in 25 % ammonia-methanol and the solution was allowed to stand at room temperature overnight. The solvent was evaporated and the residue was purified by preparative thin layer chromatography (silica gel, solvent; $CHCl_3$ : MeOH : AcOH = 85 : 15 : 3), followed by trituration with aqueous methanol to give 3.3 mg of 9-(3-O-amino-2-deoxy-β-D-*erythro*-pentofuranosyl)-guanine.
Amorphous powder,
NMR ($Me_2SO$-$d_6$ + $D_2O$): 4.3 - 4.4 (m, 1H, $C_3{'}H$).

**Claims**

1. An antiviral agent comprising a nucleoside of the formula:

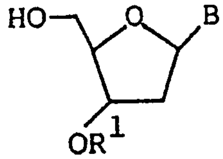

(I)

wherein B is a thymin-1-yl group, a cytosin-1-yl group, an adenin-9-yl group or a guanin-9-yl group; and $R^1$ is amino group when B being a thymin-1-yl group, a cytosin-1-yl group, an adenin-9-yl group or a guanin-9-yl group, -NHCHO when B being a thymin-1-yl group or an adenin-1-yl group, $-N=CH_2$ when B being a thymin-1-yl group, or $-N=C(CH_3)_2$ when B being a thymin-1-yl group or an adenin-9-yl group,

or a pharmaceutically acceptable salt thereof as an active therapeutic substance.

2. An agent as claimed in Claim 1, wherein the salt comprises a salt with a hydrochloric acid, a sulfuric acid, a phosphoric acid, a sulfamic acid, an acetic acid, a lactic acid, a tartaric acid, a maleic acid, a succinic acid, a methansulfonic acid., an ethanesulfonic acid, a benzenesulfonic acid or a p-toluenesulfonic acid.

3. An agent as claimed in Claim 1, wherein the compound is 9-(3-O-amino-2-deoxy-β-D-*erythro*-pentofuranoxyl)guanine.

4. A pharmaceutical composition comprising a therapeutically effective amount of a nucleoside or a salt thereof as claimed in Claim 1 or Claim 2, and a pharmaceutically acceptable carrier.

5. A pharmaceutical composition as claimed in Claim 4 in unit dosage form.

6. A pharmaceutical composition as claimed in Claim 4, wherein the compound is 9-(3-O-amino-2-deoxy-B-D-*erythro*-pentofuranoxyl)guanine.

7. A novel nucleoside represented by the formula:

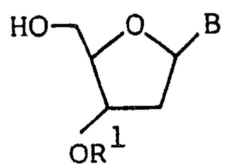

(I)

wherein B is a thymin-1-yl group, an adenin-9-yl group or a guanin-9-yl group; and $R^1$ is amino group when B being an adenin-9-yl group or a guanin-9-yl group, -NHCHO when B being a thymin-1-yl group or an adenin-1-yl group, $-N=CH_2$ when B being a thymin-1-yl group, or $-N=C(CH_3)_2$ when B being a thymin-1-yl group or an adenin-9-yl group.

8. A nucleoside as claimed in Claim 7 substantially as hereinbefore discloses in any one of the examples.

9. The use of a nucleoside of the formula:

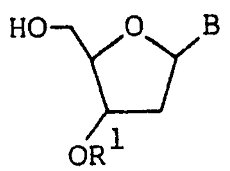

(I)

wherein B is a thymin-1-yl group, a cytosin-1-yl group, an adenin-9-yl group or a guanin-9-yl group; and $R^1$ is amino group when B being a thymin-1-yl group, a cytosin-1-yl group, an adenin-9-yl group or a guanin-9-yl group, -NHCHO when B being a thymin-1-yl group or an adenin-1-yl group, $-N=CH_2$ when B being a thymin-1-yl group, or $-N=C(CH_3)_2$ when B being a thymin-1-yl group or an adenin-9-yl group,
or a pharmaceutically acceptable salt thereof in the manufacture of a pharmaceutical preparation for the treatment of a viral disease, e.g. a retroviral infection, especially the acquired immune deficiency syndrome (AIDS) and AIDS-related complex (ARC).

10. A use of a nucleoside as claimed in Claim 9, wherein the compound is 9-(3-O-amino-2-deoxy-β-D-*erythro*-pentofuranoxyl)guanine.

European Patent Office

# EUROPEAN SEARCH REPORT

Application number

EP 90300491.9

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| D,X | NUCLEIC ACID RESEARCH, Symposium Series no. 16, 1985 K. KONDO et al. "Synthesis of 5'(3'-)O-amino nucleosides" pages 93-96 * Abstract (line 3) , page 96 formula 17d * -- | 1 | C 07 H 19/073<br>C 07 H 19/173<br>A 61 K 31/70 |
| A | DE - A1 - 3 639 780 (MAX-PLANCK-GESELLSCHAFT ZUR FÖRDERUNG DER WISSENSCHAFTEN EV) * Abstract * -- | 1,3-5, 7,9,10 | |
| A | EP - A2 - 0 287 215 (THE WELLCOME FOUNDATION) * Abstract; claims * -- | 1,4,5, 7,9,10 | |
| A | DE - A1 - 3 739 366 (BOEHRINGER MANNHEIM GMBH) * Abstract; Claims; page 6, line 25 * ---- | 1,4,5, 7,9,10 | TECHNICAL FIELDS SEARCHED (Int Cl 5)<br>C 07 H 19/00 |

The present search report has been drawn up for all claims

| Place of search | Date of completion of the search | Examiner |
|---|---|---|
| VIENNA | 17-04-1990 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document